# EUROPEAN PATENT APPLICATION

(11) **EP 3 998 274 A1**
(43) Date of publication of application: **18.05.2022**
(21) Application number: 20840389.9
(22) Date of filing: 06.07.2020
(51) Int. Cl.: C07K 1/14, C07K 1/16, G01N 27/62, C08B 37/00, G01N 1/28, G01N 30/06, G01N 30/72, G01N 30/88

(54) **PREPARATION METHOD FOR SAMPLE TO BE ANALYZED, ANALYSIS METHOD, AND KIT FOR PREPARATION OF SAMPLE TO BE ANALYZED**

(30) Priority: 12.07.2019 JP 2019130188
(71) Applicant: SHIMADZU CORPORATION, Kyoto-shi, Kyoto 604-8511 (JP)
(72) Inventor: NISHIKAZE, Takashi, Kyoto-shi, Kyoto 604-8511 (JP); FURUKAWA, Jun-ichi, Sapporo-shi, Hokkaido 060-0808 (JP); HANAMATSU, Hisatoshi, Sapporo-shi, Hokkaido 060-0808 (JP)
(74) Representative: Mewburn Ellis LLP
(86) International application number: PCT/JP2020/026489
(87) International publication number: WO 2021/010221

(57) **Abstract**

A method for preparing an analysis sample includes providing a sample containing a glycan, and performing an amidation reaction through contacting the sample with an amidation reaction solution having a pH of 7.7 or more, the amidation reaction solution containing at least one first compound which is to be reacted with a lactone included in the glycan, and which is selected from the group consisting of ammonia to be reacted with a lactone included in the glycan, primary amines having one or no carbon atom directly linked to a carbon atom linked to an amino group, hydrazine, hydrazine derivatives, hydroxylamine, and salts thereof, and alkalinizing agents to amidate the lactone, wherein when the alkalinizing agent is an amine, the alkalinizing agent is at least one amine selected from the group consisting of branched primary amines in which two or more carbon atoms are directly linked to a carbon atom linked to an amino group, secondary amines, and tertiary amines.

## Description

### TECHNICAL FIELD

The present invention relates to a method for preparing an analysis sample, an analysis method, and a kit for preparing an analysis sample.

### BACKGROUND ART

Sialic acid is a sugar existing in various forms in a living body. Sialic acid is included in glycans linked to proteins in the living body, and is often present at a non-reducing terminal of a glycan. Therefore, sialic acid is disposed in the outermost end of such a glycoprotein molecule and plays an important role because it is directly recognized by other molecules.

Sialic acids may have different linkage types with adjacent sugars. For example, α2,3- and α2,6- linkage types are primary known for human N-linked glycans (N-glycans), and in addition to these linkage types, α2,8- and α2,9- linkage types are known for O-linked glycans (O-glycans) and glycosphingolipids. Sialic acids with such different linkage types are recognized from different molecules and can play different roles.

In mass spectrometry or the like, glycans including sialic acid is subjected to modification of sialic acid as pretreatment. This neutralizes the negatively-charged carboxy group of sialic acid by esterification, amidation, or the like, to eliminate disadvantages such as suppression of ionization and detachment of sialic acid. In the lactonization of sialic acid, the stability of the produced lactone varies depending on the linkage type, and therefore the difference in stability enables linkage-specific modification and analysis of sialic acids. However, lactones are extremely unstable, easily hydrolyzed in water, and more quickly hydrolyzed under acidic or basic conditions. For this reason, it has been reported that the amidation-based stabilization of lactones formed by modification in pretreatment (see Patent Literature 1 and Non Patent Literature 1). Lactones are present in glycans in the living body, glycans of antibody drugs, and the like, and stabilization can also be performed when analyzing them. Direct amidation of lactones by aminolysis described in Non Patent Literature 2 enables quick and specific amide modification of lactones, and is expected to be used in the future.

### CITATION LIST

### PATENT LITERATURE

Patent Literature 1: JP 6135710 B

### NON PATENT LITERATURE

Non Patent Literature 1: Nishikaze T, Tsumoto H, Sekiya S, Iwamoto S, Miura Y, Tanaka K. "Differentiation of Sialyl Linkage Isomers by One-Pot Sialic Acid Derivatization for Mass Spectrometry-Based Glycan Profiling" Analytical Chemistry, (USA), ACS Publications, February 21, 2017, Volume 89, Issue 4, pp. 2353-2360
Non Patent Literature 2: Hanamatsu H, Nishikaze T, Miura N, Piao J, Okada K, Sekiya S, Iwamoto S, Sakamoto N, Tanaka K, Furukawa JI. "Sialic Acid Linkage Specific Derivatization of Glycosphingolipid Glycans by Ring-Opening Aminolysis of Lactones" Analytical Chemistry, (USA), ACS Publications, October 29, 2018, Volume 90, Issue 22, pp. 13193-13199

### SUMMARY OF INVENTION

### TECHNICAL PROBLEM

It is preferable to suppress hydrolysis of a lactone and to amidate a lactone included in a glycan more efficiently.

### SOLUTION TO PROBLEM

A first aspect of the present invention relates to a method for preparing an analysis sample, the method including: providing a sample containing a glycan; and performing an amidation reaction through contacting the sample with an amidation reaction solution having a pH of 7.7 or more, the amidation reaction solution containing at least one first compound which is to be reacted with a lactone included in the glycan, and which is selected from the group consisting of ammonia, primary amines having one or no carbon atom directly linked to a carbon atom linked to an amino group, hydrazine, hydrazine derivatives, hydroxylamine, and salts thereof, and alkalinizing agents to amidate the lactone, wherein when the alkalinizing agent is an amine, the alkalinizing agent is at least one amine selected from the group consisting of branched primary amines in which two or more carbon atoms are directly linked to a carbon atom linked to an amino group, secondary amines, and tertiary amines.

A second aspect of the present invention relates to an analysis method including preparing an analysis sample by the method for preparing an analysis sample according to the first aspect, and analyzing the analysis sample prepared.

A third aspect of the present invention relates to a kit for preparing an analysis sample used in the method for preparing an analysis sample according to the first aspect or the analysis method according to the second aspect.

### ADVANTAGEOUS EFFECTS OF INVENTION

The present invention enables more efficient amidation of a lactone included in a glycan.

### BRIEF DESCRIPTION OF DRAWINGS

Fig. 1 is a flowchart showing a procedure of an analysis method according to an embodiment.
Fig. 2 is a flowchart showing a procedure of an analysis method according to an embodiment.
Fig. 3 is a conceptual diagram showing structures of glycans produced in examples.
Fig. 4 is a mass spectrum including peaks corresponding to glycans shown in Fig. 3.
Fig. 5 is a graph showing concentrations of tert-butylamine and ratios of amidated sialic acid in an amidation reaction performed on beads.
Fig. 6 is a graph showing the types of alkalinizing agents and ratios of amidated sialic acid in an amidation reaction performed on beads.
Fig. 7 is a graph showing a composition of a solution and a ratio of amidated sialic acid in an amidation reaction performed under acidic conditions on beads.
Fig. 8 is a graph showing concentrations of methylamine hydrochloride and ratios of amidated sialic acid in an amidation reaction performed on beads.
Fig. 9 is a graph showing the types of solvent and ratios of amidated sialic acid in an amidation reaction performed on beads.
Fig. 10 is a graph showing the types of alkalinizing agents and ratios of amidated sialic acid in an amidation reaction performed in a liquid phase.
Fig. 11 shows mass spectra obtained by mass spectrometry on an analysis sample obtained by performing an amidation reaction with ethylamine hydrochloride that is not stable isotopically labeled (upper part) and ethylamine hydrochloride that is stable isotopically labeled (lower part), using tert-butylamine as an alkalinizing agent.
Fig. 12 is an enlarged view showing peaks corresponding to A3 (left side) and A3F (right side) in a mass spectrum obtained by mass spectrometry on an analysis sample obtained by mixing glycans obtained by performing an amidation reaction with ethylamine hydrochloride that is not stable isotopically labeled and ethylamine hydrochloride that is stable isotopically labeled.

### DESCRIPTION OF EMBODIMENTS

Hereinafter, embodiments for carrying out the present invention will be described with reference to the drawings. The inventors have found that a lactone contained in a glycan can be amidated more efficiently by performing an amidation reaction using an alkalinizing agent.

### - First embodiment -

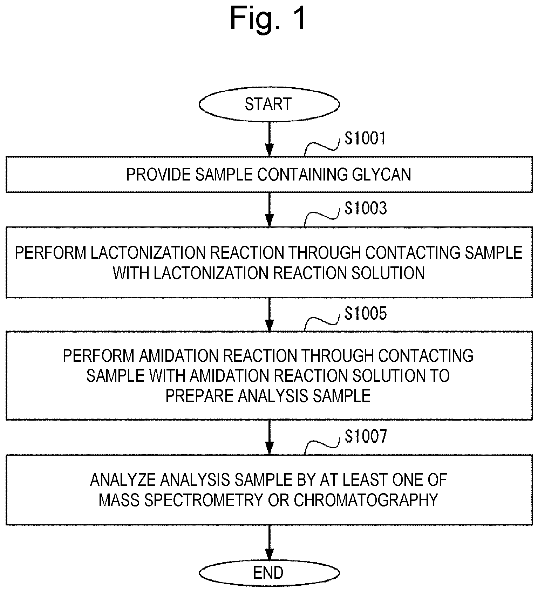

Fig. 1 is a flowchart showing a procedure of an analysis method according to a method for preparing an analysis sample of the present embodiment. In the method for preparing an analysis sample of the present embodiment, sialic acids of a glycan contained in a sample are lactonized based on linkage type, and thereafter the lactones produced are amidated. In step S 1001, a sample containing a glycan is prepared.

### (Sample)

The sample containing a glycan is not particularly limited, and can contain at least one molecule selected from the group consisting of glycans, free glycans, glycopeptides, glycoproteins, and glycolipids. In the method for preparing an analysis sample of the present embodiment, a linkage-specific modification of sialic acids included in a glycan is performed. It is preferable that the glycan in the sample include glycans that may have a sialic acid at the terminal, such as N-linked glycans, O-linked glycans, and glycolipid glycans. It is more preferable that the glycan in the sample include or may include at least one of α2,3-sialic acid, α2,8-sialic acid, and α2,9-sialic acid, and it is still more preferable that the glycan in the sample include or may include α2,6-sialic acid in addition to at least one of these sialic acids.

When the sample contains a free glycan, the glycan can be a glycan released from a glycoprotein, a glycopeptide, or a glycolipid. As the release method, a method such as enzyme treatment using N-glycosidase, O-glycosidase, endoglycoceramidase, or the like, hydrazinolysis, β elimination by alkali treatment, or the like can be used. When an N-linked glycan is released from a peptide chain of a glycopeptide and a glycoprotein, enzyme treatment with peptide-N-glycosidase F (PNGase F), peptide-N-glycosidase A (PNGase A), endo-β-N-acetylglucosaminidase (Endo M), or the like is suitably used. In addition, modification such as pyridylamination (PA labeling) of the reducing terminal of the glycan can be appropriately performed. Before the enzyme treatment, the peptide chain of the glycopeptide or glycoprotein described later may be cleaved.

When the sample contains a glycopeptide or a glycoprotein, a treatment for suppressing a side reaction of the peptide moiety can be appropriately performed as described in the section of "Suppression of side reaction of glycopeptide and glycoprotein" described later. It is preferable that those having a large number of amino acid residues in the peptide chain of the glycopeptide or glycoprotein be used by cleaving the peptide chain by enzymatic cleavage or the like. For example, in the case of preparing a sample for mass spectrometry, the number of amino acid residues of the peptide chain is preferably 30 or less, more preferably 20 or less, still more preferably 15 or less. When it is required to clarify the origin of the peptide to which the glycan is linked, the number of amino acid residues of the peptide chain is preferably 2 or more, more preferably 3 or more.

As a digestive enzyme in the case of cleaving the peptide chain of the glycopeptide or glycoprotein, trypsin, Lys-C, arginine endopeptidase, chymotrypsin, pepsin, thermolysin, proteinase K, pronase E, or the like is used. Two or more of these digestive enzymes may be used in combination. The conditions for cleaving the peptide chain are not particularly limited, and an appropriate protocol according to the digestive enzyme to be used is adopted. Before this cleavage, denaturation treatment or alkylation treatment of the proteins and peptides in the sample may be performed. The conditions for the denaturation treatment or the alkylation treatment are not particularly limited. The peptide chain may be cleaved not by enzymatic cleavage but by chemical cleavage or the like.

Upon completion of step S1001, the process proceeds to step S1003.

### (Lactonization reaction)

In step S1003, a lactonization reaction is performed through contacting the sample with a reaction solution for lactonization (hereinafter referred to as lactonization reaction solution) to lactonize at least a part of sialic acids contained in a glycan (hereinafter, "lactonization reaction" refers to the lactonization reaction in step S1003 unless otherwise specified). Hereinafter, an example in which a part of sialic acids is lactonized in a linkage-specific manner using a lactonization reaction solution and another part of sialic acids are modified differently from lactonization will be described. However, modification different from lactonization does not have to be performed. In the lactonization reaction, α2,3-sialic acid, α2,8-sialic acid, and α2,9-sialic acid are suitably lactonized.

The lactonization reaction solution contains a dehydration-condensation agent and a first reactant containing an alcohol, an amine, or a salt thereof. The first reactant is a reactant for performing modification by esterification or amidation by linking at least a part of the first reactant to sialic acids. The first reactant functions as, for example, a nucleophile. The type and concentration of the dehydration-condensation agent and the first reactant are adjusted so as to selectively cause a dehydration reaction or a modification reaction by esterification or amidation based on the linkage type of sialic acids.

When modification different from lactonization by the first reactant is not performed, the lactonization reaction solution may contain a dehydration-condensation agent and does not have to contain the first reactant.

Linkage-specific lactonization will be described. The lactone produced by intramolecular dehydration of the carboxy group of α2,3-sialic acid is a six-membered ring, and the lactone produced by intramolecular dehydration of the carboxy group of α2,6-sialic acid is a seven-membered ring. Therefore, α2,3-sialic acid that produces a six-membered ring more stable than a seven-membered ring is more likely to be lactonized than α2,6-sialic acid. Because the carboxy group of α2,3-sialic acid is at a position where steric hindrance is relatively large as compared with the carboxy group of α2,6-sialic acid, a large molecule hardly reacts with α2,3-sialic acid as compared with α2,6-sialic acid. Based on such a difference in the molecular structure due to the linkage type of sialic acids, the kind and concentration of the dehydration-condensation agent and the first reactant are adjusted so as to be modified differently depending on the linkage type of sialic acids.

### (Dehydration-condensation agent in lactonization reaction)

The dehydration-condensation agent preferably contains carbodiimide. This is because when carbodiimide is used, a carboxy group present at a site where steric hindrance is large is less likely to be amidated than when a phosphonium-based dehydration-condensation agent (so-called BOP reagent) or an uronium-based dehydration-condensation agent is used as the dehydration-condensation agent. Examples of the carbodiimide include N,N'-dicyclohexylcarbodiimide (DCC), N-(3-dimethylaminopropyl)-N'-ethylcarbodiimide (EDC), N,N'-diisopropylcarbodiimide (DIC), 1-tert-butyl-3-ethylcarbodiimide (BEC), N,N'di-tert-butylcarbodiimide, 1,3-di-p-tolylcarbodiimide, bis(2,6-diisopropylphenyl)carbodiimide, bis(trimethylsilyl)carbodiimide, 1,3-bis(2,2-dimethyl-1,3-dioxolane-4-ylmethyl)carbodiimide (BDDC), and salts thereof.

### (Additive in lactonization reaction)

To promote the dehydration condensation by the dehydration-condensation agent and suppress the side reaction, it is preferable to use a high nucleophilic additive in addition to carbodiimide. As the highly nucleophilic additive, 1-hydroxybenzotriazole (HOBt), 1-hydroxy-7-aza-benzotriazole (HOAt), 4-(dimethylamino)pyridine (DMAP), ethyl 2-cyano-2-(hydroxyimino)acetate (Oxyma), N-hydroxy-succinimide (HOSu), 6-chloro-1-hydroxybenzotriazole (Cl-HoBt), N-hydroxy-3,4-dihydro-4-oxo-1,2,3-benzotriazine (HOOBt) and the like are preferably used.

### (Reactant in lactonization reaction (first reactant))

The amine used as the first reactant preferably contains a primary or secondary alkylamine containing two or more carbon atoms. The primary alkylamine is preferably ethylamine, propylamine, isopropylamine, butylamine, sec-butylamine, tert-butylamine, or the like. The secondary alkylamine is preferably dimethylamine, ethylmethylamine, diethylamine, propylmethylamine, isopropylmethylamine, or the like. It is preferable to use an amine having a branched alkyl group such as isopropylamine from the viewpoint of making it difficult for a carboxy group present at a site where steric hindrance is large, such as the carboxy group of α2,3-sialic acid, to be amidated. When an amine is used as the first reactant in the lactonization reaction solution, a carboxy group of a part of sialic acids such as α2,6-sialic acid is amidated based on the linkage type of sialic acids.

The alcohol used as the first reactant is not particularly limited, and for example, methanol, ethanol, or the like can be used. When an alcohol is used as the reactant of the lactonization reaction solution, a carboxy group of a part of sialic acids such as α2,6-sialic acid is esterified based on the linkage type of sialic acids.

The first reactant may contain salts of the above-described amine and alcohol.

### (Concentration of dehydration-condensation agent and amine)

The concentration of the dehydration-condensation agent in the lactonization reaction solution is, for example, preferably 1 mM to 5 M (hereinafter, M represents mol/L), and more preferably 10 mM to 3 M. When carbodiimide and a highly nucleophilic additive such as HOAt or HOBt are used in combination, each concentration is preferably in the above range. The concentration of the amine in the lactonization reaction solution is preferably 0.01 to 20 M, more preferably 0.1 M to 10 M. The reaction temperature in the lactonization reaction is preferably about -20°C to 100°C, and more preferably -10°C to 50°C.

### (Phase for lactonization reaction)

The lactonization reaction can be performed in either a liquid phase or a solid phase. As long as the sample and the lactonization reaction solution can be contacted with each other, the state of the sample when the lactonization reaction is caused is not particularly limited.

When the reaction is performed in a solid phase, a solid-phase carrier can be used without particular limitation as long as it can immobilize a glycan, a glycopeptide, a glycoprotein, or the like. For example, to immobilize a glycopeptide or a glycoprotein, a solid-phase carrier having an epoxy group, a tosyl group, a carboxy group, an amino group, or the like as a ligand can be used. To capture a glycan, a solid-phase carrier having a hydrazide group, an aminooxy group, or the like as a ligand can be used. From the viewpoint of ionization efficiency and the like, it is also preferable to cause a carrier for hydrophilic interaction chromatography (hereinafter referred to as HILIC), that is, a stationary phase to adsorb a glycan, and it is more preferable that the carrier for HILIC contain an amide group.

The reaction in a state where the sample is immobilized on a solid-phase carrier enables easier removal of the reaction solution and desalting purification and simple preparation of the sample. When a solid-phase carrier is used, the sample is immobilized in the form of a glycoprotein or a glycopeptide, and after the lactonization reaction, the sample after the lactonization reaction is cleaved by a glycosidase such as PNGase F, or the like, whereby the sample after the lactonization reaction can be recovered as a free glycan.

The sample after the lactonization reaction may be subjected to treatment such as purification, desalting, and solubilization by a known method or the like as necessary. The same applies before and after the amidation reaction described later.

For the release of the sample from the solid-phase carrier, the conditions described for the amidation reaction described later can be adopted. The reaction in a state where the sample is immobilized on a solid-phase carrier enables easier removal of the lactonization reaction solution after the lactonization reaction or the like and efficient modification of sialic acids.

Upon completion of step S1003, the process proceeds to step S1005.

### (Amidation reaction)

In step S1005, the sample is contacted with a reaction solution (hereinafter referred to as amidation reaction solution), and an amidation reaction for amidating the sialic acids lactonized in step S1003 is performed to obtain an analysis sample. The inventors have found a method for quickly and directly amidating a lactone, which is completely different from common technical knowledge in which a lactone is ring-opened by hydrolysis and then a carboxy group is amidated. Because this reaction is suitably carried out even under anhydrous conditions, it is a reaction different from hydrolysis, and is considered to be aminolysis based on an interaction between an amino group and a lactone. Hereinafter, the ring opening and amidation of a lactone with ammonia, an amine, or a salt thereof, which is possible even under anhydrous conditions, is referred to as aminolysis. Because this aminolysis reaction does not substantially require a dehydration-condensation agent, it is possible to selectively amidate only lactonized sialic acids without affecting normal sialic acids in which no lactone is formed. In the following embodiments, an example in which an amidation reaction by aminolysis is performed will be described except for amidation in a lactonization reaction and a nonspecific modification reaction described later.

In the following embodiments, when referring to a lactone for sialic acid as in "a lactone formed in a sialic acid", the lactone formed inside a sialic acid is also referred to in addition to a lactone formed between a sialic acid and a monosaccharide adjacent to the sialic acid.

The amidation reaction solution contains a reactant containing ammonia, an amine, or a salt thereof (hereinafter referred to as second reactant), and an alkalinizing agent (alkalinizing reagent). The second reactant is an amidation reactant for performing modification by amidation by linking at least a part of the second reactant to sialic acids. The second reactant is, for example, a nucleophile. The alkalinizing agent is a compound for increasing the pH of the amidation reaction solution. Preferably, the amidation reaction is carried out only by contacting the sample with the amidation reaction solution, and the lactone is stabilized by a simple operation.

The amidation reaction does not require a dehydration-condensation agent, but the amidation reaction solution may contain a dehydration-condensation agent. For example, the amidation reaction solution may be prepared by adding ammonia, an amine, or a salt thereof, and an alkalinizing agent without removing the lactonization reaction solution added to the sample in step S1003. As described above, in the amidation reaction, the lactone can be stabilized by a simple operation. In the amidation reaction, it is described that an operation of reacting the sample with a dehydration-condensation agent after contacting the sample with the amidation reaction solution is not performed, but the amidation reaction may be performed, for example, for other purposes.

When the lactonization reaction solution contains the first reactant, the second reactant contained in the amidation reaction solution is different from the first reactant. When the analysis sample obtained by the method for preparing an analysis sample of the present embodiment is analyzed by mass spectrometry, the second reactant is selected such that the first reactant and the second reactant are different in mass. The first reactant and the second reactant are selected according to the mass resolution of mass spectrometry such that the obtained two kinds of modified products are subjected to mass separation with high accuracy. The first reactant and the second reactant may be different kinds of substances, or may be the same kind of substances having different masses depending on stable isotopes. In addition, an isobaric tag represented by iTRAQ may be used. In this case, because the tag is designed so that m/z of product ions obtained by cleavage performed between the mass spectrometry in the first stage and the mass spectrometry in the second stage is different, identification of the linkage type of sialic acid and the lactone form can be performed by mass spectrometry in two or more stages such as tandem mass spectrometry (MS/MS). As described above, when the modified product modified with each of the first reactant and the second reactant is subjected to mass spectrometry in two or more stages, it is only required that these modified products can be separated at different m/z in any stage. When the analysis sample obtained by the method for preparing an analysis sample of the present embodiment is analyzed by chromatography, it is preferable that the first reactant and the second reactant have different substituents in order to facilitate separation from each other by chromatography.

### (Amine in amidation reaction)

In the following embodiments, the term "amine" includes hydrazine, hydrazine derivatives, and hydroxylamine, and does not include ammonia or salts of ammonia. When an amine is used in the amidation reaction, the amine contained in the second reactant is at least one compound selected from a primary amine in which one or no carbon atom is directly linked to a carbon atom linked to an amino group, hydrazine, a hydrazine derivative, a hydroxylamine, and salts thereof. As described above, in the case of the primary amine, even when the carbon chain has a branch, if the branch is present at a position away from the amino group, a decrease in the efficiency of the amidation reaction is suppressed, which is preferable.

The second reactant is more preferably a primary amine having a linear hydrocarbon group, and still more preferably a primary amine having a linear alkyl group. The second reactant is preferably a primary amine having 10 or less carbon atoms as a primary amine having a linear alkyl group, more preferably a primary amine having 6 or less carbon atoms, that is, methylamine, ethylamine, propylamine, butylamine, pentylamine, and hexylamine, and most preferably methylamine. The amine contained in the amidation reaction solution preferably has a linear structure having no branches (hereinafter, "branch" indicates branch of a hydrocarbon chain) or has a small number of carbon atoms because the lactone is more efficiently amidated.

The hydrazine derivative contained in the second reactant is not particularly limited. In the following embodiments, hydrazides such as acetohydrazide, acetic acid hydrazide, benzohydrazide, and benzoic acid hydrazide are also included in the hydrazine derivative, and can be used as the second reactant. The hydrazine derivative contained in the second reactant can be at least one compound selected from the group consisting of methylhydrazine, ethylhydrazine, propylhydrazine, butylhydrazine, phenylhydrazine, benzylhydrazine, acetohydrazide, acetic acid hydrazide, benzohydrazide, and benzoic acid hydrazide. Hydrazine or a derivative thereof as the second reactant is preferably hydrazine or methylhydrazine from the viewpoint of increasing or maintaining the efficiency of the amidation reaction.

When the second reactant is a primary amine having an unsaturated chain hydrocarbon group, the unsaturated chain hydrocarbon group preferably contains a double link, the unsaturated chain hydrocarbon group more preferably contains an allyl group, and the amine is still more preferably allylamine. The second reactant may be a primary amine containing a hydroxy group, and in this case, ethanolamine is preferable. The amine contained in the amidation reaction solution may contain various functional groups other than the alkyl group. When the glycan is modified so as to contain such a functional group as a result of the amidation reaction, the glycan subjected to the modification is more easily separated not only by mass spectrometry but also by chromatography or the like.

The second reactant can be a salt of ammonia or the amine described above as the second reactant. Compounds with altered properties such as mass, such as stable isotopes, and compounds with specific modifications to have altered function are often sold or distributed in the form of salts. Therefore, using these salts as the second reactant enables more flexible or appropriate selection of the analysis method. In addition, when a salt is used, it may be difficult to increase the pH of the amidation reaction solution, but in the method for preparing an analysis sample of the present embodiment, because an alkalinizing agent is used, this disadvantage can also be solved.

Examples of the salt of ammonia or amine contained in the second reactant include an inorganic acid salt or an organic acid salt of ammonia or amine, and an inorganic salt such as carbonate salt, hydrochloride salt, nitrate salt, sulfate salt, phosphate salt, or methanesulfonate salt is preferable, carbonate salt, hydrochloride salt, or nitrate salt is more preferable, and hydrochloride salt is still more preferable. As the hydrochloride salt of the primary amine having a linear hydrocarbon group, methylamine hydrochloride salt, ethylamine hydrochloride salt, propylamine hydrochloride salt, butylamine hydrochloride salt, or pentylamine hydrochloride salt is more preferable, and methylamine hydrochloride salt is still more preferable.

Even when the second reactant is not a salt, amidation can be more efficiently performed by increasing the pH with an alkalinizing agent.

### (Alkalinizing agent in amidation reaction)

The alkalinizing agent contained in the amidation reaction solution is not particularly limited as long as it is a compound that increases the pH of the amidation reaction solution. However, when the alkalinizing agent is an amine, there is a possibility that the alkalinizing agent is linked to sialic acids by an amidation reaction similarly to the second reactant. In this case, a peak of a mass spectrum or a chromatogram is split at the time of mass spectrometry or chromatography described later, which is not preferable. Therefore, when the alkalinizing agent is an amine, it is preferable that the alkalinizing agent is less likely to link to sialic acids than the second reactant. For example, the amine contained in the alkalinizing agent can be an amine having a steric hindrance larger than that of the second reactant. From this viewpoint, when the alkalinizing agent is an amine, the alkalinizing agent is at least one amine selected from the group consisting of branched primary amines in which two or more carbon atoms are directly linked to a carbon atom linked to an amino group, secondary amines, and tertiary amines. Here too, "branched" indicates that the carbon chain has a branch.

Examples of the branched primary amine can include isopropylamine, secondary butylamine (sec-butylamine), tertiary butylamine (tert-butylamine), 2-hexanamine, and 1,3-dimethylamylamine. The branched primary amine is preferably a molecule having 3 to 7 carbon atoms, more preferably isopropylamine, sec-butylamine, or tert-butylamine, from the viewpoint of easy availability, easy handling, solubility, reactivity, water solubility, or the like. The alkalinizing agent may contain one primary amine, or may contain two or more primary amines in any ratios.

The secondary amine included in the alkalinizing agent can be a dialkylamine, a secondary cyclic alkylamine, or a secondary aromatic amine. The dialkylamine preferably has a total carbon number of 1 to 10, more preferably 1 to 6, and can be, for example, dimethylamine, diethylamine, or dibutylamine. The secondary cyclic alkylamine preferably has a total carbon number of 1 to 10, more preferably 4 to 10, and can be, for example, piperidine or 2,2,6,6-tetramethylpiperidine. As in morpholine, an atom other than carbon or hydrogen may be used for a part of the cyclic structure. The secondary aromatic amine preferably has a total carbon number of 7 to 20, more preferably 7 to 15, and can be, for example, methylaniline, phenylmethylamine, or diphenylamine. The secondary amine is preferably a dialkylamine, more preferably dimethylamine, diethylamine, or dibutylamine, from the viewpoint of easy availability, easy handling, solubility, reactivity, water solubility, or the like. The alkalinizing agent may contain one secondary amine, or may contain two or more secondary amines in any ratios.

The tertiary amine included in the alkalinizing agent can be a trialkylamine, a tertiary cyclic alkylamine, or a tertiary aromatic amine. The trialkylamine preferably has a total carbon number of 1 to 10, more preferably 1 to 6, and can be, for example, trimethylamine, triethylamine, or tributylamine. The tertiary cyclic alkylamine preferably has a total carbon number of 1 to 10, more preferably 4 to 10, and can be, for example, N-methylpiperidine. As in N-methylmorpholine, an atom other than carbon or hydrogen may be used for a part of the cyclic structure. The tertiary aromatic amine preferably has a total carbon number of 8 to 20, more preferably 8 to 15, and can be, for example, dimethylaniline, diphenylmethylamine, or triphenylamine. The tertiary amine is preferably trialkylamine or N-methylmorpholine, more preferably trimethylamine, triethylamine, or tributylamine, from the viewpoint of easy availability, easy handling, solubility, reactivity, water solubility, or the like. The alkalinizing agent may contain one tertiary amine, or may contain two or more secondary amines in any ratios.

As an example, the alkalinizing agent can include at least one selected from the group consisting of quaternary ammonium cations, tertiary amines, secondary amines, primary amines in which two or more carbon atoms are directly linked to a carbon atom linked to an amino group, hydroxides of alkali metals, hydroxides of alkaline earth metals, hydroxides of tetraalkylammonium, guanidine, guanidine derivative, salts thereof, and alkaline buffer solutions.

The quaternary ammonium cation contained in the alkalinizing agent is not particularly limited, and can be, for example, a tetraalkylammonium cation, a tetraalkylphosphonium cation, an imidazolium cation, a pyrazolium cation, a pyridinium cation, or a pyrimidinium cation. The alkalinizing agent may contain one quaternary ammonium cation, or may contain two or more quaternary ammonium cations in any ratios.

The hydroxide contained in the alkalinizing agent is not particularly limited as long as it is a hydroxide of an alkali metal, a hydroxide of an alkaline earth metal, or a hydroxide of tetraalkylammonium. As the hydroxide of an alkali metal, at least one selected from the group consisting of calcium hydroxide (Ca(OH)2), lithium hydroxide (LiOH), sodium hydroxide (NaOH), potassium hydroxide (KOH), rubidium hydroxide (RbOH), and cesium hydroxide (CsOH) can be suitably used. As the hydroxide of an alkaline earth metal, at least one selected from the group consisting of strontium hydroxide (Sr(OH)2), barium hydroxide (Ba(OH)2), europium hydroxide (II) (Eu(OH)2), and thallium hydroxide (I) (TIOH) can be suitably used. As the hydroxide of tetraalkylammonium, at least one selected from the group consisting of tetramethylammonium hydroxide (N(CH3)4OH) and tetraethylammonium hydroxide (N(C2H5)4OH) can be suitably used. The alkalinizing agent may contain one hydroxide, or may contain two or more hydroxides in any ratios.

The guanidine derivative contained in the alkalinizing agent is not particularly limited, and can be, for example, 1,1,3,3-tetramethylguanidine or 1,1,3,3-tetraethylguanidine. The alkalinizing agent may contain one guanidine derivative, or may contain two or more guanidine derivatives in any ratios.

Examples of the salt of the branched primary amine, secondary amine, tertiary amine, guanine, and guanine derivative contained in the alkalinizing agent include an inorganic acid salt and an organic acid salt, and an inorganic salt such as carbonate salt, hydrochloride salt, nitrate salt, sulfate salt, phosphate salt, or methanesulfonate salt is preferable.

The kind of the alkaline buffer solution contained in the alkalinizing agent is not particularly limited, and for example, it can be at least one selected from the group consisting of Tris buffer solutions, Good buffer solutions, borate buffer solutions, and carbonate buffer solutions.

In the following embodiments, when it is described that "an amidation reaction solution is added to a sample", "a sample is contacted with an amidation reaction solution", or the like, the following first and second cases are included. The first case is a case where a solution containing the second reactant and a solution containing an alkalinizing agent are separately prepared, and both solutions are added one after the other to a sample and mixed. The second case is a case where an amidation reaction solution containing the second reactant and an alkalinizing agent is prepared and added to a sample. The same applies to the above-described lactonization reaction solution.

### (Concentration of amidation reaction solution)

The concentration of the second reactant in the amidation reaction solution is not particularly limited, and is preferably 0.1 M (M is mol/l) or more, more preferably 0.3 M or more, still more preferably 0.5 M or more, still more preferably 1.0 M or more, and most preferably 3.0 M or more. As a suitable example, the amidation reaction solution contains ammonia or the primary amine described above, particularly methylamine, and the concentration of ammonia or the primary amine such as methylamine is preferably 0.1 M or more, more preferably 0.3 M or more, still more preferably 0.5 M or more, further preferably 1.0 M or more, and most preferably 3.0 M or more. The higher the concentration of the second reactant in the amidation reaction solution, the more reliably the lactone can be amidated. The concentration of the alkalinizing agent in the amidation reaction solution is not particularly limited as long as the pH of the amidation reaction solution can be set to a desired value or more, and the alkalinizing agent does not adversely affect the accuracy of analysis by linking to sialic acids as a modified product different from the second reactant. As an example, the concentration of the alkalinizing agent, in particular, the concentration of tert-butylamine is preferably 0.10% or more, more preferably 0.30% or more, still more preferably 1% or more, and further preferably 3% or more in terms of volume/volume% from the viewpoint of efficiently causing amidation. The concentration of the alkalinizing agent, in particular, the concentration of tert-butylamine can be appropriately 80% or less, 60% or less, or the like.

### (Solvent of amidation reaction solution)

The solvent of the amidation reaction solution is preferably an aqueous solvent or a mixed solvent of an aqueous solvent and an organic solvent from the viewpoint of reliably causing amidation. The solvent of the amidation reaction solution can be, for example, water, methanol, ethanol, dimethyl sulfoxide (DMSO), or an acetonitrile aqueous solution.

### (pH of amidation reaction solution)

The pH of the amidation reaction solution is 7.7 or more. The pH of the amidation reaction solution is preferably 8.0 or more, more preferably 8.8 or more, still more preferably 10.3 or more. When the pH of the amidation reaction solution increases, side reactions such as hydrolysis are suppressed, and lactones are more reliably amidated using various second reactants, which is preferable.

### (Time for causing amidation reaction)

The amidation reaction is complete within seconds to minutes. Therefore, the time during which the sample is in contact with the amidation reaction solution to amidate the lactone by the amidation reaction (hereinafter referred to as reaction time) is preferably less than 1 hour, more preferably less than 30 minutes, still more preferably less than 15 minutes, still more preferably less than 5 minutes, and most preferably less than 1 minute. Preferably, the sample may be washed with the amidation reaction solution, or the sample held by a carrier or the like may be passed through the solution. The time during which the sample is in contact with the amidation reaction solution is not particularly limited, and can be appropriately 0.1 seconds or more, 1 second or more, or the like from the viewpoint of sufficiently completing the reaction, or the like. The sample and the amidation reaction solution may be mixed and dried as it is without providing a reaction time. As described above, because the amidation reaction is completed in a short time, it is possible to suppress degradation of an unstable lactone and impairment of quantitativity in analysis of glycans. In addition, by setting the reaction time of the amidation reaction to be short, the sample can be analyzed more efficiently.

### (Phase in which amidation reaction is performed)

As long as the sample and the amidation reaction solution can be contacted with each other, the state of the sample when the amidation reaction is caused is not particularly limited, and may be a solid phase or a liquid phase. When the amidation reaction is performed in a liquid phase, the amidation reaction solution may be added to the sample while leaving the solution after the lactonization reaction as described above, or known pretreatment such as purification, desalting, and solubilization may be performed after the lactonization reaction. When the amidation reaction is performed in a state of being immobilized on a solid phase, the sample subjected to the lactonization reaction in the solid phase may be maintained in a state of being immobilized on the solid phase to perform the amidation reaction. Alternatively, the sample may be subjected to the lactonization reaction and then immobilized on a solid phase to perform the amidation reaction.

When the amidation reaction is performed in a solid phase, the same solid-phase carrier as that described above for the lactonization reaction can be used. For immobilization of the sample on the solid-phase carrier, the conditions described above for the lactonization reaction can be used. When the amidation reaction is performed in a solid phase, the amidation reaction solution is allowed to act on the sample immobilized on the solid-phase carrier to perform amidation, and then the sample may be released from the carrier by a chemical method, an enzymatic reaction, or the like and recovered. For example, a glycoprotein or glycopeptide immobilized on a carrier may be enzymatically cleaved by a digestive enzyme such as a glycosidase including PNGase F or trypsin and recovered, or a glycan linked to a solid-phase carrier having a hydrazide group may be released by a weakly acidic solution and recovered. In HILIC, an amidation reaction is performed with an amidation reaction solution using acetonitrile or the like as a solvent, and the sample can be eluted with an aqueous solution such as water.

In the analysis sample obtained by the preparation method described above, when the first reactant is included in the lactonization reaction solution, a sialic acid having a linkage type that is hardly lactonized, such as α2,6-sialic acid, is modified with the first reactant in the lactonization reaction. A sialic acid having a linkage type that is easily lactonized, such as α2,3-, α2,8-, or α2,9-sialic acids, is lactonized in a lactonization reaction and modified with the second reactant in the amidation reaction.

Upon completion of step S1005, the process proceeds to step S1007.

In step S1007, the analysis sample is analyzed by at least one of mass spectrometry and chromatography.

The glycans modified in each reaction by the lactonization reaction and the amidation reaction are different in mass. Therefore, it is possible to separate these glycans by mass spectrometry based on the linkage type of sialic acids.

The ionization method in mass spectrometry is not particularly limited, and matrixassisted laser desorption/ionization (MALDI) method, electrospray (ESI) method, nanoelectrospray ionization (nano-ESI) method, or the like can be used. The MALDI method is particularly preferable as the ionization method. In ionization in mass spectrometry, either a positive ion mode or a negative ion mode may be used. The mass spectrometry may be performed by single mass spectrometry or multistage mass spectrometry, whereby the structure of a glycan or the structure of a peptide chain having a linkage type other than the linkage type of sialic acids can be suitably analyzed. As the mass spectrometer, at least one or more of any mass spectrometers such as a quadrupole type, an ion trap type, and a timeof-flight type can be used in combination. Dissociation of ions, addition of atoms or atomic groups to ions, and the like can also be appropriately performed.

When analysis is performed by chromatography, liquid chromatography is preferable. The column used for liquid chromatography is not particularly limited, and a hydrophobic reversed-phase column such as C30, C18, C8, or C4, a carbon column, a normal-phase column for HILIC, or the like can be appropriately used. After liquid chromatography is performed, it is preferable to perform measurement by mass spectrometry in order to precisely analyze components in a sample by a plurality of times of separation. In this case, it is more preferable that the eluate from the liquid chromatograph is ionized by ESI or the like directly in a mass spectrometer by online control using a liquid chromatograph-mass spectrometer (LC-MS).

Data obtained by mass spectrometry or chromatography is analyzed, and sialic acids in the glycan contained in the sample are analyzed. In this data analysis, estimation or the like of the structure of glycans including the linkage type of sialic acids can be performed. A method for analyzing data obtained by mass spectrometry or chromatography is not particularly limited.

When step S1007 ends, the process ends.

### (Suppression of side reaction of glycopeptide and glycoprotein)

When the sialic acids are modified as described above by adding the lactonization reaction solution and the amidation reaction solution to the glycopeptide or glycoprotein, side reactions such as intramolecular dehydration condensation may occur between the amino group and carboxy group at the terminal of the peptide backbone and side chain of the amino acid contained in the glycopeptide or glycoprotein. In this case, there is a problem that the peak of mass spectrum corresponding to the glycan to be analyzed is separated, and analysis becomes difficult.

The inventors have clarified that the side reaction of the peptide moiety is mainly derived from the presence of an amino group, and have clarified that the side reaction of the peptide moiety can be suppressed at the time of sialic acid modification by blocking the amino group first by chemical modification or the like before sialic acid modification. For details, see the following literature: Takashi Nishikaze, Sadanori Sekiya, Shinichi Iwamoto, Koichi Tanaka. "A Universal Approach to linkage-Specific Derivatization for Sialic Acids on Glycopeptides," Journal of The American Society for Mass Spectrometry, June 2017, Volume 28, Issue 1 Supplement, poster number MP091. The modification by the lactonization reaction or the like according to the present embodiment can also be used for a glycopeptide and a glycoprotein in the same manner. For example, a reaction of blocking an amino group such as dimethyllabeling or guanidinylation is performed on a glycopeptide or a glycoprotein, and a lactonization reaction and an amidation reaction are performed. At this time, the linkage type of sialic acids can also be identified by using a method for forming a lactone according to the linkage type of sialic acids.

Some glycopeptides are less likely to cause side reactions due to the characteristics based on the amino acid sequence. For example, a glycopeptide produced by digesting an Fc region of IgG with a digestive enzyme such as trypsin does not have lysine, and the N-terminal amino group is also rapidly cyclized and dehydrated in the presence of a dehydration-condensation agent to be pyroglutamylated. As a result, no amino groups are present, and therefore no prior blocking of amino groups is necessary, such as dimethylamidation or guanidinylation. For such a glycopeptide, it is possible to obtain a mass spectrum sufficient for analysis by performing a lactonization reaction and an amidation reaction without blocking amino groups.

### (Kit for preparing analysis sample)

A kit for preparing an analysis sample (hereinafter referred to as preparation kit) suitably used in the method for preparing an analysis sample of the present embodiment is provided. The preparation kit can include a reagent, any consumable used for mass spectrometry other than the reagent, or a document in which a protocol for preparing the analysis sample in the present embodiment, a URL of a website in which the protocol is described, or the like is described. For example, the preparation kit can include an alkalinizing agent in an amidation reaction or a solution containing an alkalinizing agent. Preparing an analysis sample using the preparation kit enables more efficient preparation of an analysis sample.

### - Second embodiment -

The method for preparing an analysis sample of the second embodiment performs an amidation reaction similar to that in the method for preparing an analysis sample of the first embodiment, but is different from the first embodiment in that a lactone originally contained in a sample is amidated by an amidation reaction without performing a lactonization reaction.

### (Sample)

The sample is not particularly limited, and the same sample as that of the first embodiment can be used. It is known that lactones are present in ganglioside, which is a glycolipid, and a milk oligosaccharide chain. The polysialic acid structure that is well expressed in a brain includes sialic acid having a linkage type of α2,8- or α2,9-, and it is said that these sialic acids are likely to form lactones. It is known that glycans of biopharmaceuticals, particularly antibody drugs, contain lactones. For example, to quantify the lactone present in such a sample or to check whether the lactone is present in an unknown sample, the method for preparing an analysis sample of the present embodiment can be used.

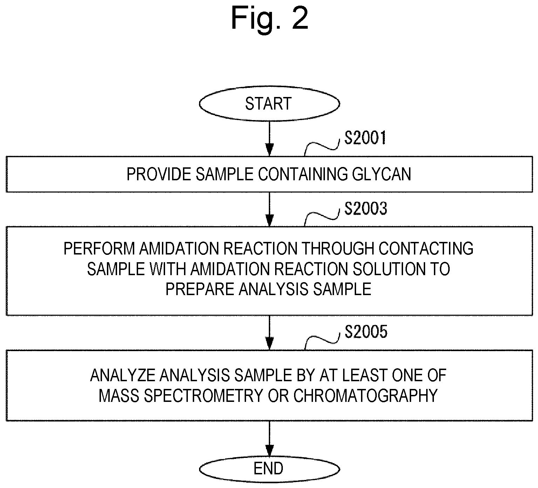

Fig. 2 is a flowchart showing a procedure of an analysis method according to the method for preparing an analysis sample of the present embodiment. Step S2001 is the same as step S1001 in the above-described embodiment, and thus description thereof is omitted. Upon completion of step S2001, the process proceeds to step S2003.

In step S2003, the sample is contacted with an amidation reaction solution to perform an amidation reaction for amidating sialic acids originally contained in the sample, thereby acquiring an analysis sample. The amidation reaction can be performed under the same conditions using the same amidation reaction solution as in the above-described embodiment. Upon completion of step S2003, the process proceeds to step S2005. Step S2005 is the same as step S1007 in the above-described embodiment, and thus description thereof is omitted.

After performing the amidation reaction in step S2003, a lactonization reaction and a further amidation reaction in the above-described embodiment may be performed. The amidation reaction performed before the lactonization reaction is referred to as first amidation reaction, and the amidation reaction performed after the lactonization reaction is referred to as second amidation reaction. In the first amidation reaction, the lactonization reaction, and the second amidation reaction, it is preferable to vary the kind of the reactant to be linked to sialic acids. In this case, the lactone originally contained in the sample, α2,3-sialic acid, α2,8-sialic acid, and α2,9-sialic acid, which have not been lactonized originally in the sample, and α2,6-sialic acid can be modified with different modified products. This enables analysis of the sialic acid lactone originally contained in the sample and linkage-specific analysis of sialic acids.

Alternatively, after the amidation reaction is performed in step S2003, a reaction for amidating or esterifying sialic acids in a manner nonspecific to linkage type (hereinafter referred to as nonspecific modification reaction) may be performed. In this case, the sample subjected to the amidation reaction is contacted with a reaction solution for nonspecific modification reaction (hereinafter referred to as nonspecific modification reaction solution), and the sialic acids contained in the sample before the amidation reaction in step S2003 and on which lactone is not formed, are modified. In the nonspecific modification reaction, each sialic acid that is contained in the sample and not amidated is modified regardless of the linkage type of sialic acid, and an analysis sample is obtained.

The nonspecific modification reaction solution contains a dehydration-condensation agent and a third reactant containing an alcohol, ammonia, an amine, or a salt thereof. The third reactant is a reactant for performing modification of esterification or amidation of sialic acids by linking at least a part of the third reactant to sialic acids in a manner nonspecific to linkage type. The third reactant functions as, for example, a nucleophile. The third reactant is selected such that the modified product obtained by the modification with the second reactant and the third reactant is separately detected by mass spectrometry or chromatography as described above. As the dehydration-condensation agent in the nonspecific modification reaction, a phosphonium-based dehydration-condensation agent or an uronium-based dehydration-condensation agent used in the second reaction of Patent Literature 1 described above can be used. As the third reactant in the nonspecific modification reaction, the amine used in the "second reaction" of Patent Literature 1 described above or an alcohol such as methanol or ethanol can be used. An esterifying agent such as 1-methyl-3-p-tolyltriazene or 1-ethyl-3-p-tolyltriazene can also be used for the nonspecific esterification reaction. When such an esterifying agent is used, a dehydration-condensation agent is not required.

In the nonspecific modification reaction, the kind of the reaction, the composition of the reaction solution, or the like is not particularly limited as long as a modification different from the modification in the amidation reaction in step S2003 can be performed on sialic acids contained in the glycan.

### (Aspect)

It is understood by those skilled in the art that the plurality of exemplary embodiments described above are specific examples of the aspects below.

(Clause 1) A method for preparing an analysis sample according to an aspect is a method for preparing an analysis sample, the method including: providing a sample containing a glycan; and performing an amidation reaction through contacting the sample with an amidation reaction solution having a pH of 7.7 or more, the amidation reaction solution containing at least one first compound which is to be reacted with a lactone included in the glycan, and which is selected from the group consisting of ammonia, primary amines having one or no carbon atom directly linked to a carbon atom linked to an amino group, hydrazine, hydrazine derivatives, hydroxylamine, and salts thereof, and alkalinizing agents to amidate the lactone, wherein when the alkalinizing agent is an amine, the alkalinizing agent is at least one amine selected from the group consisting of branched primary amines in which two or more carbon atoms are directly linked to a carbon atom linked to an amino group, secondary amines, and tertiary amines. This enables more efficient amidation of lactones contained in a glycan.

(Clause 2) A method for preparing an analysis sample according to another aspect is the method for preparing an analysis sample according to clause 1, wherein the alkalinizing agent contains at least one compound selected from the group consisting of quaternary ammonium cations, tertiary amines, secondary amines, branched primary amines in which two or more carbon atoms are directly linked to a carbon atom linked to an amino group, hydroxides of alkali metals, hydroxides of alkaline earth metals, hydroxides of tetraalkylammonium, guanidine, guanidine derivatives, salts thereof, and alkaline buffer solutions, and the alkaline buffer solution is at least one selected from the group consisting of Tris buffer solutions, Good buffer solutions, borate buffer solutions, and carbonate buffer solutions. This enables more reliable increase in the pH of the amidation reaction solution and amidation of lactones contained in a glycan

(Clause 3) A method for preparing an analysis sample according to another aspect is the method for preparing an analysis sample according to clause 1 or 2, wherein the first compound is a carbonate salt, a hydrochloride salt, a nitrate salt, a sulfate salt, a phosphate salt, or a methanesulfonate salt of at least one compound selected from ammonia, amine, hydroxylamine, hydrazine, and a hydrazine derivative. This enables more flexible or appropriate selection of the analysis method.

(Clause 4) A method for preparing an analysis sample according to another aspect is the method for preparing an analysis sample according to any one of clauses 1 to 3, wherein the amidation reaction is performed only by contacting the sample with the amidation reaction solution. This enables easy preparation of an analysis sample.

(Clause 5) A method for preparing an analysis sample according to another aspect is the method for preparing an analysis sample according to any one of clauses 1 to 4, wherein the amidation reaction solution does not contain a dehydration-condensation agent to be reacted with the lactone. This enables easier preparation of the amidation reaction solution.

(Clause 6) A method for preparing an analysis sample according to another aspect is the method for preparing an analysis sample according to any one of clauses 1 to 5, wherein in the amidation reaction, after the sample is contacted with the amidation reaction solution, an operation of reacting the sample with a dehydration-condensation agent is not performed. This enables reduction of the number of steps in preparing the analysis sample, making the preparation simpler.

(Clause 7) A method for preparing an analysis sample according to another aspect is the method for preparing an analysis sample according to any one of clauses 1 to 6, wherein a time during which the sample is in contact with the amidation reaction solution to perform the amidation reaction is shorter than 30 minutes. This enables efficient preparation of an analysis sample in a shorter time.

(Clause 8) A method for preparing an analysis sample according to another aspect is the method for preparing an analysis sample according to any one of clauses 1 to 7, wherein the first compound contains a linear hydrocarbon group. This enables more reliable modification of sialic acids with the first compound.

(Clause 9) A method for preparing an analysis sample according to another aspect is the method for preparing an analysis sample according to clause 8, wherein the linear hydrocarbon group is an alkyl group. The first compound enables further reliable modification of sialic acids.

(Clause 10) A method for preparing an analysis sample according to another embodiment is the method for preparing an analysis sample according to clause 9, wherein a carbon number of the linear hydrocarbon group is any one of 1 to 6. This enables still further reliable modification of sialic acids with the first compound.

(Clause 11) A method for preparing an analysis sample according to another aspect is the method for preparing an analysis sample according to any one of clauses 1 to 10, wherein the lactone is formed in sialic acids of the glycan. This enables accurate analysis of lactones of sialic acids which are unstable and difficult to analyze.

(Clause 12) A method for preparing an analysis sample according to another aspect is the method for preparing an analysis sample according to any one of clauses 1 to 10, wherein the lactone is formed in at least one of α2,3-sialic acid, α2,8-sialic acid, and α2,9-sialic acid of the glycan. This enables linkage-specific analysis or the like of the sialic acid lactones or sialic acids originally contained in the sample.

(Clause 13) A method for preparing an analysis sample according to another aspect is the method for preparing an analysis sample according to clause 11 or 12, further including contacting the sample prepared with a lactonization reaction solution containing a dehydration-condensation agent to be reacted with the sialic acids to lactonize at least a part of the sialic acids. This enables analysis of a part of sialic acids and another part of sialic acids separately based on the stability of lactone.

(Clause 14) A method for preparing an analysis sample according to another aspect is the method for preparing an analysis sample according to clause 13, wherein the lactonization reaction solution further contains a reactant to be linked to the sialic acids contained in the glycan, and the reactant is different in mass from the first compound, and the sample is contacted with the lactonization reaction solution, a part of the sialic acids are lactonized based on a linkage type of the sialic acids, and at least a part of the reactant is linked to another part of the sialic acids. This enables analysis of a part of sialic acids and another part of sialic acids separately based on the linkage type of sialic acids.

(Clause 15) A method for preparing an analysis sample according to another aspect is the method for preparing an analysis sample according to clause 14, wherein the sample is contacted with the lactonization reaction solution to lactonize α2,3-sialic acid, α2,8-sialic acid, or α2,9-sialic acid and to link a part of the reactant to α2,6-sialic acid. This enables analysis of α2,6-sialic acid separately from α2,3-sialic acid, α2,8-sialic acid, and α2,9-sialic acid.

(Clause 16) An analysis method according to an aspect includes preparing an analysis sample by the method for preparing an analysis sample according to any one of clauses 1 to 15, and analyzing the analysis sample prepared. This enables more efficient amidation of lactones contained in a glycan.

(Clause 17) A method for preparing an analysis sample according to another aspect is the method for preparing an analysis sample according to clause 16, wherein the sample prepared is analyzed by at least one of mass spectrometry and chromatography. This enables separation and analysis of glycans based on the modification performed at the time of preparing an analysis sample.

(Clause 18) A kit for preparing an analytical sample according to an aspect is used in the method for preparing an analysis sample according to any one of clauses 1 to 15 or the analysis method according to clause 16 or 17. This enables efficient preparation of an analysis sample.

The present invention is not limited to the contents of the above embodiments. Other aspects conceivable within the scope of the technical idea of the present invention are also included within the scope of the present invention.

### EXAMPLE

Hereinafter, examples according to the embodiments will be described. The present invention is not limited to the following examples. In the following description, % indicates volume/volume% unless otherwise specified.

### (First to fifth examples and comparative example)

### <Preparation of glycan sample for evaluation>

A glycan called A2 glycan (33A2) containing two α2,3-sialic acids was produced by the following method. To a tube containing 20 µL of 1 nmol/µL of α2,3-sialylglycopeptide (α2,3-SGP; FUSHIMI Pharmaceutical Co., Ltd.) PNGase F (SIGMA) for 2.5 U was added, and the mixture was incubated at 37°C overnight to release N-glycan called A2 glycan (33A2). The released glycans were desalted with a Stage Tip Carbon.

A sample was obtained by redissolving the desalted glycans in appropriate water. When an amidation reaction or the like was performed on beads, this sample was directly reacted with beads. When an experiment was performed as an in-solution reaction in a liquid phase in a tube, the solution was dispensed into an Eppendorf tube, and the solvent was further removed with SpeedVac (Thermo Fisher Scientific) to prepare a sample.

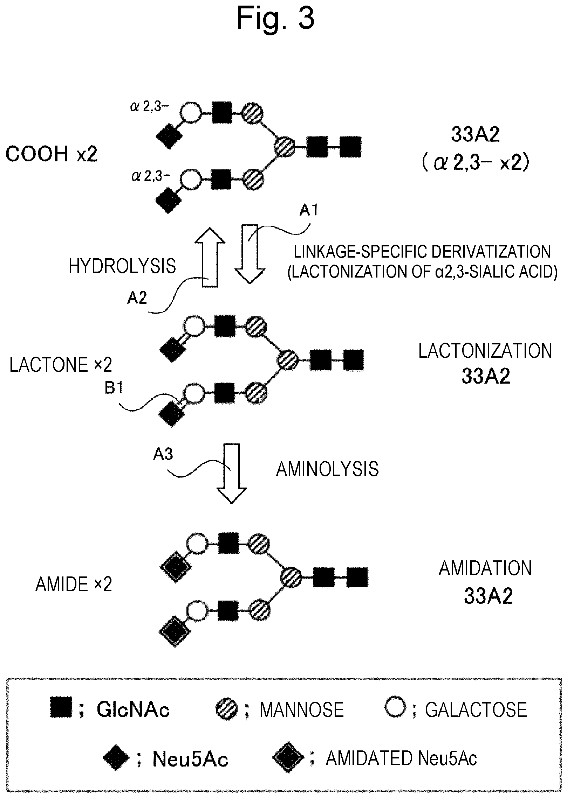

Fig. 3 is a diagram schematically showing the structure of glycans in samples used in the following examples and assumed changes in the structure. The glycan 33A2 has a basic structure composed of N-acetyl-D-glucosamine (GlcNAc) and mannose (Man), and two antennae. GlcNAc, galactose (Gal), and sialic acid (Neu5Ac) are linked to each of the two antennae. A lactone was formed at a linking portion between α2,3-sialic acid at the non-reducing terminal and galactose to which the sialic acid was linked, and this point was indicated by a double line B1. The glycan 33A2 is lactonized by linkage-specific modification of sialic acid using a lactonization reaction solution (arrow A1). When this lactone is hydrolyzed, it returns to its original sialic acid structure (arrow A2). This lactone can be amidated directly by aminolysis (arrow A3).

### <Basic reaction conditions in each of the following examples>

In the first to fifth examples and a comparative example below, some lactones are not amidated by aminolysis and remain as lactones in some cases. However, lactones are unstable and thus may affect the quantitativity of experimental results. Therefore, in the following experiment, an amidation reaction was performed as aminolysis (referred to as primary aminolysis), and then aminolysis (secondary aminolysis) was performed again with an amidation reaction solution containing an amine different from the amine used in the primary aminolysis. In this way, the lactone that did not react in the primary aminolysis was converted into an amide different from the amide generated in the primary aminolysis, thereby performing quantitative evaluation. Hereinafter, methylamine hydrochloride salt was used for the primary aminolysis, and ethylamine was used for the secondary aminolysis. Therefore, in a mass spectrum, a peak corresponding to a methylamidated glycan can be read as a peak corresponding to a glycan amidated by primary aminolysis, and a peak corresponding to an ethylamidated glycan can be read as a peak corresponding to a glycan remaining as a lactone after primary aminolysis and quantitatively evaluated.

### <Basic reaction conditions when aminolysis is performed on beads>

A sample containing a glycan was adsorbed to hydrazide beads (manufactured by Sumitomo Bakelite Co., Ltd., according to the standard protocol of BlotGlyco), and capping of excess hydrazide groups was performed. Thereafter, a lactonization reaction solution (2 M isopropylamine (iPA) hydrochloride salt, 500 mM EDC-HCl, 500 mM HOBt) for performing modification in a manner specific to linkage type of sialic acid was added to the sample, and the mixture was stirred for 1 hour to lactonize α2,3-sialic acid (at this time, α2,6-sialic acid was isopropylamidated if it was present). Subsequently, the beads were washed three times with 200 µL of methanol (MeOH), and 100 µL of a primary aminolysis solution (components will be described in each of the following examples because they may be different) was added to the beads, lightly stirred, and centrifuged to remove the primary aminolysis solution. Next, the beads were sequentially washed with MeOH (200 µL x 3), H2O (200 µL x 3), and MeOH (200 µL x 3), a 17.5% ethylamine aqueous solution as a secondary aminolysis solution was added thereto, and the mixture was lightly stirred, and then centrifuged to remove the solution. Further, the beads were washed with H2O (200 µL x 3), and glycans were released from the beads according to the standard protocol of BlotGlyco, and then measurement was performed by mass spectrometry described later.

### <Basic reaction conditions when aminolysis is performed in liquid phase>

To a tube containing a sample containing dried and solidified glycans, 20 µL of a lactonization reaction solution for performing the above-described linkage-specific modification of sialic acid was added, and the mixture was stirred for 1 hour to lactonize α2,3-sialic acid. Thereafter, 20 µL of a primary aminolysis solution composed of a 3M methylamine hydrochloride salt aqueous solution was added thereto, and the mixture was uniformly mixed by vortexing. Thereafter, 5 µL of a base as an alkalinizing agent was added, and the mixture was uniformly mixed by vortexing. Subsequently, acetonitrile (ACN) containing 2.5% trifluoroacetic acid (TFA) was added to return the pH to the vicinity of neutrality, and amide purification was performed in HILIC mode using GL-Tip Amide manufactured by GL Science, excess reagents were removed, and then the eluted glycans were evaporated to dryness with SpeedVac. Thereafter, to amidize the remaining lactone, a 17.5% ethylamine aqueous solution was added to perform secondary aminolysis, the mixture was evaporated to dryness with SpeedVac, and measurement by mass spectrometry described later was performed.

### <Conditions of mass spectrometry>

All measurements by mass spectrometry were performed by on-target 3AQ labeling. The dried glycans were well redissolved in 10 µL of water. To a µfocus plate (Hudson Surface Technology, Inc.), 0.5 µL was added dropwise, and 0.5 µL of 100 mM 3AQ/CA (3-aminoquinoline/p-coumaric acid) and 2 mM ADP (ammonium dihydrogen phosphate) dissolved in 50% ACN were added as a matrix, and the mixture was reacted together with the plate on a heat block at 75°C for 1.5 hours (the reducing terminals of the glycans were labeled with 3AQ). After completion of the reaction, the plate was returned to room temperature, and measurement was performed in negative ion mode using MALDI-QIT-TOF-MS (AXIMA-Resonance, Shimadzu/Kratos).

### <Evaluation method in data analysis>

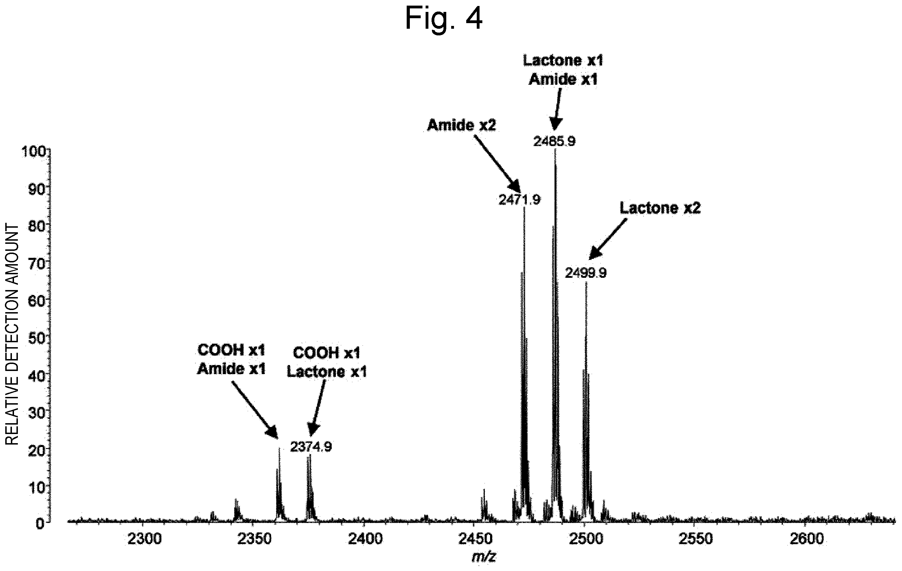

Fig. 4 is a diagram showing an example of a mass spectrum of a sample after performing the secondary aminolysis to 33A2 containing sialic acid in any of an unmodified state (COOH), an amidated state (Amide), and a lactonized state (Lactone) after performing the primary aminolysis reaction. The horizontal axis represents m/z (corresponding to the mass-to-charge ratio), and the vertical axis represents the relative detection amount.

Because 33A2, which is the sample used for the evaluation, contains two sialic acids, it is first lactonized by linkage-specific modification (iPA formation), but after the subsequent primary aminolysis, each of them can take a hydrolyzed carboxylic acid type (COOH), an amide type (Amide) in which the sialic acid has undergone aminolysis, and a lactone type (Lactone) in which the sialic acid remains as a lactone, and therefore several peaks can be detected in the mass spectrum by a combination thereof. Here, to accurately evaluate the amount of sialic acids remaining as lactone after aminolysis, one more aminolysis (secondary aminolysis) is performed using ethylamine to convert the sialic acid into ethylamide. Because the conditions for the secondary aminolysis are conditions under which the lactone is completely ethylamidated, the ethylamidated form can be read as a lactone form. When hydrolysis does not occur and the lactone can be converted to a 100% methylamidated form by the primary aminolysis, a peak is obtained only at m/z 2471.9.

In the evaluation of the ratio of the COOH form, the Amide form, and the Lactone form, considering that two sialic acids were contained in one molecule, the relative intensity of each peak was read from the mass spectrum, the peak intensity of each of the COOH form, the Amide form, and the Lactone form was calculated, and the whole was normalized to 100%.

### (First example)

### <Study on tert-butylamine concentration as alkalinizing agent (on beads)>

An experiment was performed according to the basic reaction conditions for aminolysis on beads described above. As an amidation reaction solution for the primary aminolysis, 3M methylamine hydrochloride salt containing 0 to 50% tert-butylamine was used. Here, tert-butylamine is an alkalinizing agent, and 3M methylamine hydrochloride salt is a substance (second reactant) constituting a modified product in amidation.

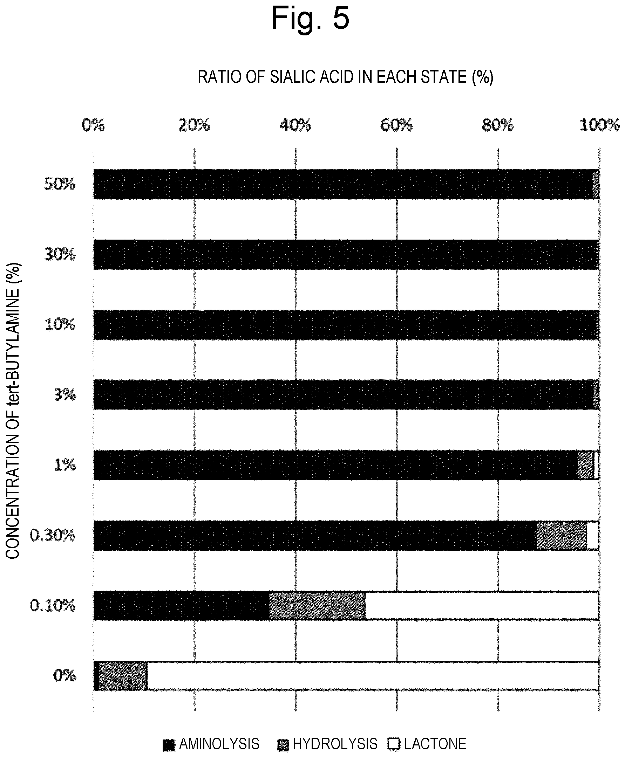

Fig. 5 is a graph showing the concentration (volume/volume%) of tert-butylamine and the ratio of amidated sialic acids in the amidation reaction of this example. When tert-butylamine was not contained, aminolysis was confirmed, but most of sialic acids remained as a lactonized form, and some sialic acids were hydrolyzed. When tert-butylamine was added even in a small amount, aminolysis occurred, and almost 100% methylamidation occurred at a concentration of tert-butylamine of about 10%. As a result, it was found that it is possible to promote aminolysis by increasing the pH of the amidation reaction solution with another base when an amine hydrochloride salt is used.

### (Second example)

### <Study on kind of alkalinizing agent (on beads)>

An experiment was performed according to the basic reaction conditions for aminolysis on beads described above. As an amidation reaction solution for performing the primary aminolysis, a 3M methylamine hydrochloride salt solution containing any one of either 10% or 1M of various alkalinizing agents (10% aqueous ammonia, 10% isopropylamine (iPA), 10% tert-butylamine (t-BA), 10% dimethylamine (DMA), 10% trimethylamine (TMA), 10% methylmorpholine (NMM), 10% tetramethylguanidine (TMG), 1M sodium hydroxide solution (NaOH)) was used.

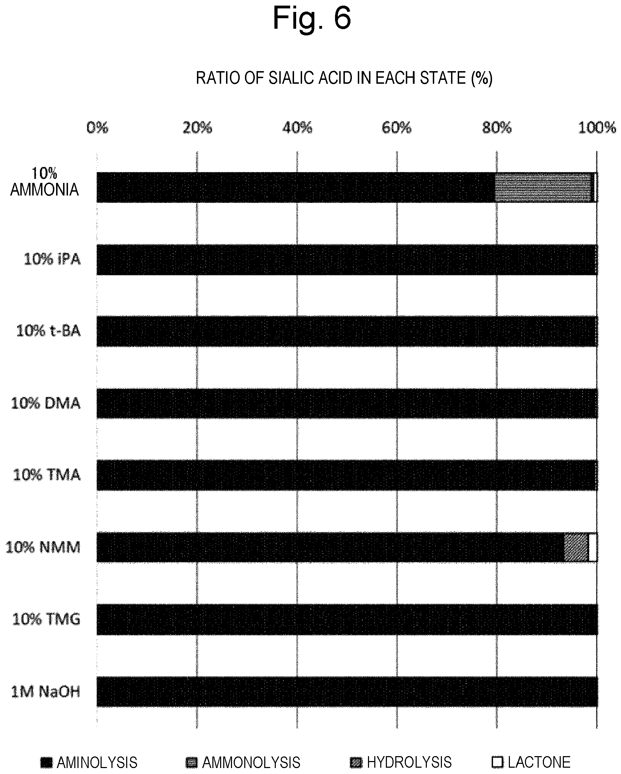

Fig. 6 is a graph showing the kind of alkalinizing agent and the ratio of amidated sialic acids in the amidation reaction of this example. From Fig. 6, it is found that it is possible to cause aminolysis regardless of the base used as the alkalinizing agent, and it is possible to promote aminolysis regardless of the kind of alkalinizing agent.

### (Comparative example)

### <Study on aminolysis under acid conditions (on beads)>

An experiment was performed according to the basic reaction conditions for aminolysis on beads described above. As an amidation reaction solution for performing the primary aminolysis, a 3M methylamine hydrochloride salt (MA-HCl) solution containing an acid (0.1% or 2% trifluoroacetic acid (TFA)) instead of a base was used.

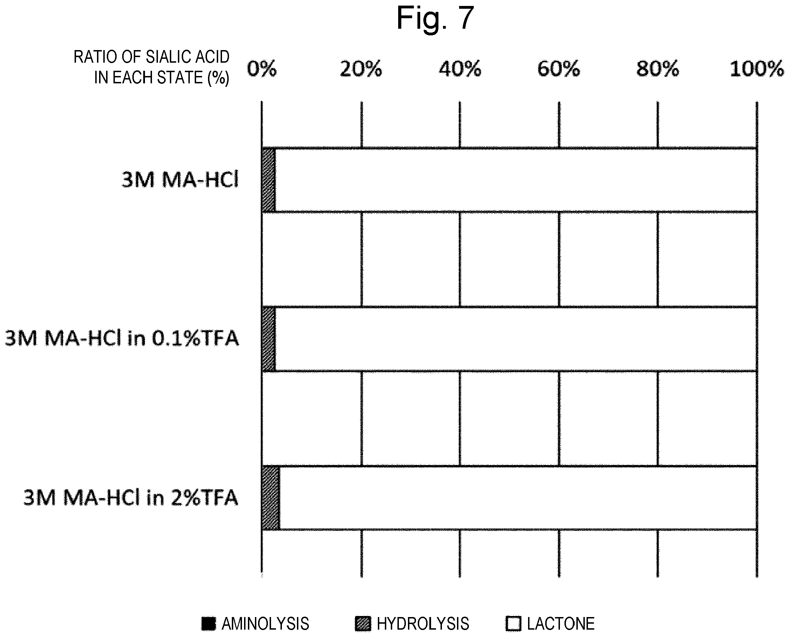

Fig. 7 is a graph showing the composition of the solution and the ratio of amidated sialic acids in the amidation reaction of this comparative example. From Fig. 7, it was found that under the condition in which trifluoroacetic acid was added instead of a base to lower the pH of the amidation reaction solution, the result was consistent with the case in which no acid was added, and aminolysis did not proceed at all even when 3M MA-HCl, which is an amine hydrochloride salt of high concentration, was used.

### (Third example)

### <Study on concentration of amine hydrochloride salt as second reactant (on beads)>

An experiment was performed according to the basic reaction conditions for aminolysis on beads described above. As an amidation reaction solution for performing the primary aminolysis, 10 mM to 3 M of a methylamine hydrochloride salt solution containing 10% tert-butylamine was used.

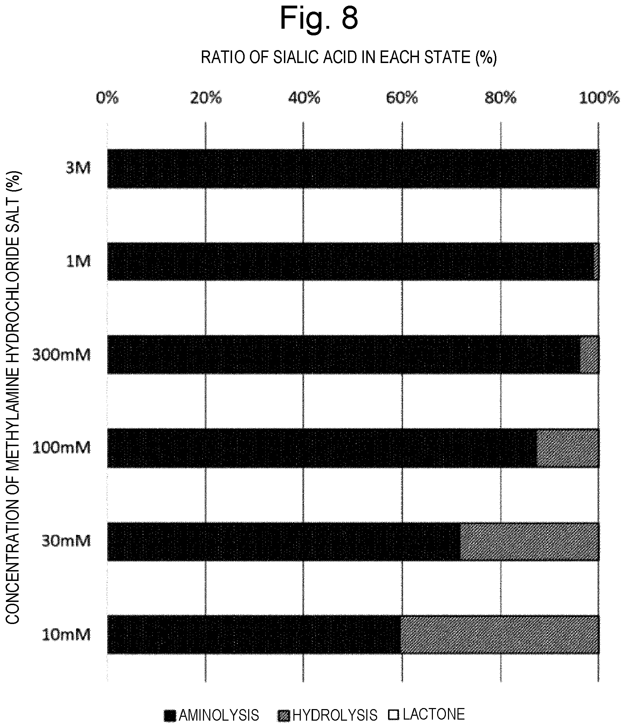

Fig. 8 is a graph showing the concentrations of methylamine hydrochloride salt and the ratios of amidated sialic acids in the amidation reaction of this example. From Fig. 8, it was found that aminolysis and hydrolysis occur in competition, but the concentration on the amine hydrochloride salt side is preferably higher.

### (Fourth example)

### <Study on solvent (on beads)>

An experiment was performed according to the basic reaction conditions for aminolysis on beads described above. As an amidation reaction solution for performing the primary aminolysis, various solutions (solvent: water (H2O), methanol (MeOH), ethanol (EtOH), dimethyl sulfoxide (DMSO), 80% acetonitrile (ACN) aqueous solution) containing 10% of tert-butylamine (t-BA) and 100 mM of methylamine hydrochloride salt were used.

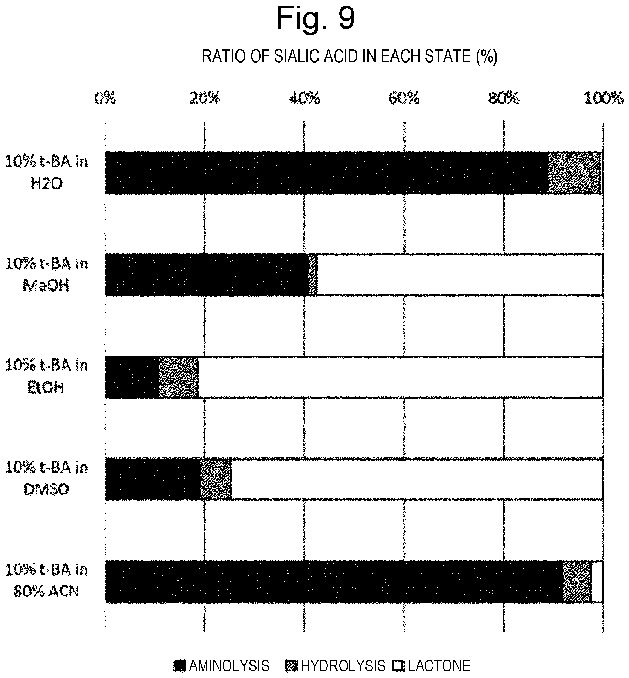

Fig. 9 is a graph showing the kinds of solvent and the ratios of amidated sialic acids in the amidation reaction of this example. From Fig. 9, it was found that an aqueous solvent is preferable from the viewpoint of efficiently causing aminolysis, but aminolysis proceeds without any problem even when an organic solvent is partially mixed.

### (Fifth example)

### <Study on kind of alkalinizing agent (liquid phase)>

An experiment was performed according to the basic reaction conditions for aminolysis in a liquid phase described above. As an alkalinizing agent contained in the amidation reaction solution, tert-butylamine (t-BA), a 50% dimethylamine aqueous solution (DMA), a 28% trimethylamine aqueous solution (TMA), tetramethylguanidine (TMG), or a 1 M sodium hydroxide (NaOH) aqueous solution was used.

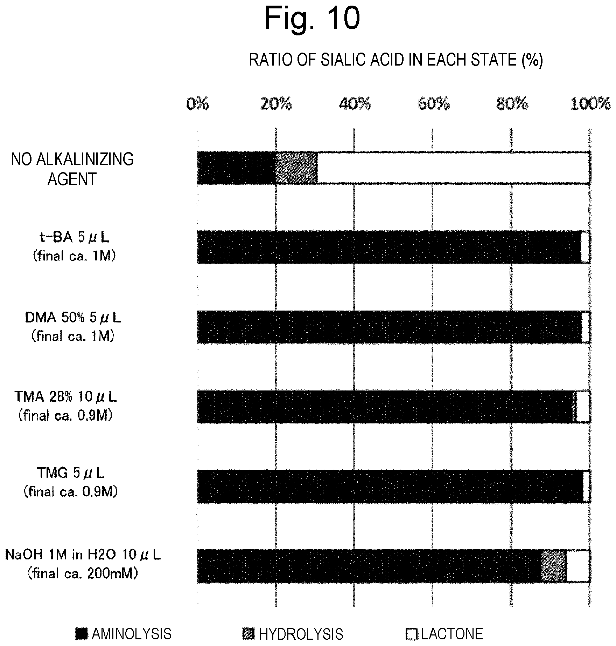

Fig. 10 is a graph showing the kinds of alkalinizing agent and the ratios of amidated sialic acids in the amidation reaction of this example. From Fig. 10, it was found that when the amidation reaction was performed in a liquid phase (in solution), the proportion of sialic acids in which aminolysis occurred remained at a certain level when an alkalinizing agent was not added, but it was possible to promote aminolysis by adding an alkalinizing agent.

The order of adding the solution containing the second reactant and the solution containing the alkalinizing agent to the glycan having a lactonized sialic acid was also studied. As a result, the solution containing the second reactant may be added and then the solution containing the alkalinizing agent may be added, and even when the solution containing the second reactant and the solution containing the alkalinizing agent are mixed and added to the glycan, the same result as described above was obtained.

### (Sixth example)

### <Study on aminolysis using stable isotopically labeled amine hydrochloride salt>

N-glycans were released from human serum-derived α-anti-trypsin (AAT) using PNGase F. The released N-glycans were linked to hydrazide beads according to the protocol of BlotGlyco manufactured by Sumitomo Bakelite Co., Ltd. Thereafter, capping of excess hydrazide groups was performed according to the protocol of BlotGlyco. A lactonization reaction solution (2 M isopropylamine (iPA) hydrochloride, 500 mM EDC-HCl, 500 mM HOBt) for modifying glycans on beads in a manner specific to linkage type of sialic acid was added to 100 µL beads, and α2,6-sialic acid was isopropylamidated and α2,3-sialic acid was lactonized while slowly stirring at 800 rpm for 1 hour. Thereafter, the beads were washed three times with 200 µL of MeOH, and then 100 µL of an ethylamine hydrochloride salt (EtNH2-HCl) solution or a stable isotopically labeled ethylamine hydrochloride salt (EtNH2-d5-HCl, both at 3 M concentration) solution, to which 10% tert-butylamine was added as an alkalinizing agent, was added to the beads, and the mixture was gently stirred and centrifuged to remove the amidation reaction solution. Further, the beads were washed three times with 200 µL of MeOH and three times with 200 µL of water, and thereafter, were subjected to high sensitivity labeling for MALDI-MS according to the BlotGlyco protocol, and glycans were recovered, and mass spectra were acquired in positive ion mode using 2,5-dihydroxybenzoic acid. Here, MALDI-MS refers to mass spectrometry in which ionization is performed by MALDI.

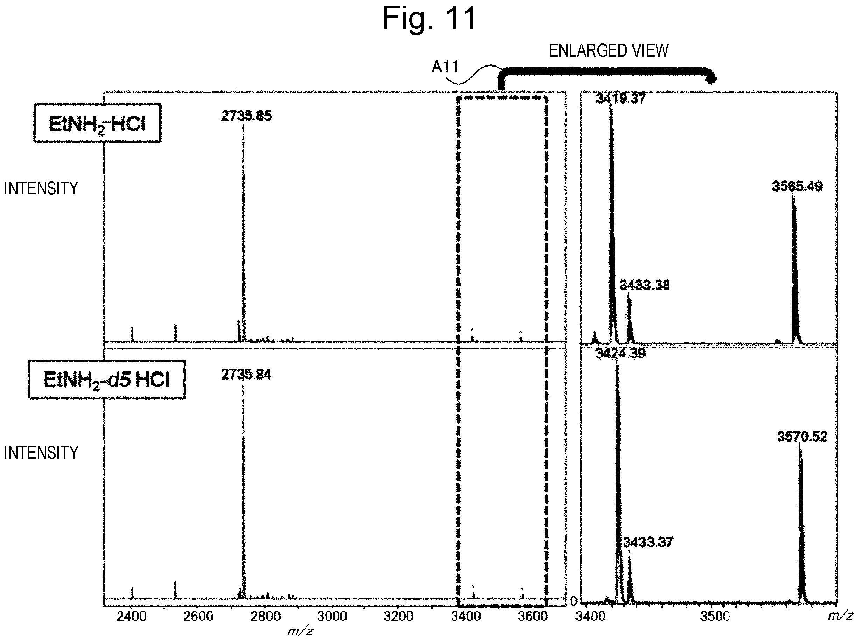

Fig. 11 is a diagram showing mass spectra obtained in this example. The horizontal axis represents m/z, and the vertical axis represents the intensity at the time of detection. In Fig. 11, the upper part is a mass spectrum when the amidation reaction was performed using an ethylamine hydrochloride salt that was not stable isotopically labeled, and the lower part is a mass spectrum when the amidation reaction was performed using an ethylamine hydrochloride salt that was stable isotopically labeled. In the upper and lower parts, an enlarged view of the mass spectrum on the left side is shown on the right side (arrow A11). The peaks observed at m/z 2735.8 were A2-type glycans in which two α2,6-sialic acids were added, and these were converted to isopropylamide at the time of linkage-specific modification of sialic acid, and therefore did not react with subsequent aminolysis, and were observed at the same m/z even when ethylamine hydrochloride EtNH2-d5-HCl that was stable isotopically labeled was used. On the other hand, the peak at 3419.4 observed when ethylamine hydrochloride EtNH2-HCl that was not stable isotopically labeled was used was a triantennary A3-type glycan containing one α2,3-sialic acid and two α2,6-sialic acids, but a shift of 5Da (m/z 3565.5 to 3570.5) was observed when ethylamine hydrochloride EtNH2-d5-HCl that was stable isotopically labeled was used. These results indicate that even isotopically labeled primary amines, which are usually available only in hydrochloride salt, can promote aminolysis by forcibly increasing the pH using an alkalinizing agent.

### (Seventh example)

In the same manner as in the sixth example, glycans modified using each of ethylamine hydrochloride that was not isotopically labeled and ethylamine hydrochloride that was stable isotope-labeled were mixed in equal amounts to prepare samples for mass spectrometry. The prepared samples for mass spectrometry were subjected to mass spectrometry in the same manner as in the sixth example.

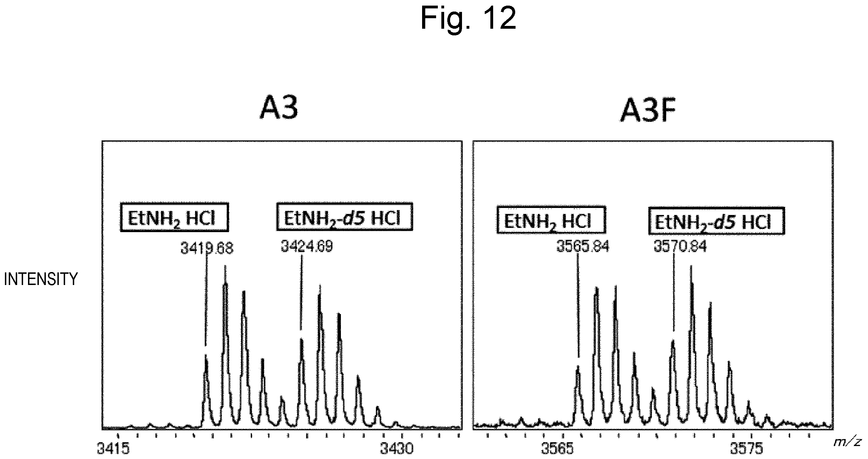

Fig. 12 is an enlarged view of mass spectra obtained in this example. In Fig. 12, peaks corresponding to A3 (left side in the figure) and A3F (right side in the figure), which are representative α2,3-sialic acid-containing triple-chain glycans contained in AAT, are shown. The peaks of A3 and A3F are each observed to be shifted by 5Da by stable isotope labeling, but it was found that the ionic intensities are almost the same when mixed in equal amounts. This means that the ionization efficiency does not change depending on the presence or absence of a stable isotope labeling after aminolysis, and it indicates that even when a stable isotope-labeled amine hydrochloride salt is used, application to comparative mass spectrometry analysis is possible by forcibly increasing the pH to promote aminolysis.

The disclosures of the following priority application (1) and literature (2) referred to herein are incorporated herein by reference.
(1) Japanese Patent Application No. 2019-130188 (filed on July 12, 2019)
(2) Takashi Nishikaze, Sadanori Sekiya, Shinichi Iwamoto, Koichi Tanaka. "A Universal Approach to linkage-Specific Derivatization for Sialic Acids on Glycopeptides," Journal of The American Society for Mass Spectrometry, June 2017, Volume 28, Issue 1 Supplement, poster number MP091

## Claims

1. A method for preparing an analysis sample, the method comprising:
providing a sample containing a glycan; and
performing an amidation reaction through contacting the sample with an amidation reaction solution having a pH of 7.7 or more, the amidation reaction solution containing at least one first compound which is to be reacted with a lactone included in the glycan, and which is selected from the group consisting of ammonia, primary amines having one or no carbon atom directly linked to a carbon atom linked to an amino group, hydrazine, hydrazine derivatives, hydroxylamine, and salts thereof, and alkalinizing agents to amidate the lactone,
wherein when the alkalinizing agent is an amine, the alkalinizing agent is at least one amine selected from the group consisting of branched primary amines in which two or more carbon atoms are directly linked to a carbon atom linked to an amino group, secondary amines, and tertiary amines.

2. The method for preparing an analysis sample according to claim 1, wherein
the alkalinizing agent contains at least one compound selected from the group consisting of quaternary ammonium cations, tertiary amines, secondary amines, branched primary amines in which two or more carbon atoms are directly linked to a carbon atom linked to an amino group, hydroxides of alkali metals, hydroxides of alkaline earth metals, hydroxides of tetraalkylammonium, guanidine, guanidine derivatives, salts thereof, and alkaline buffer solutions, and
the alkaline buffer solution is at least one selected from the group consisting of Tris buffer solutions, Good buffer solutions, borate buffer solutions, and carbonate buffer solutions.

3. The method for preparing an analysis sample according to claim 1 or 2, wherein
the first compound is a carbonate salt, a hydrochloride salt, a nitrate salt, a sulfate salt, a phosphate salt, or a methanesulfonate salt of at least one compound selected from ammonia, amine, hydroxylamine, hydrazine, and a hydrazine derivative.

4. The method for preparing an analysis sample according to claim 1 or 2, wherein
the amidation reaction is performed only by contacting the sample with the amidation reaction solution.

5. The method for preparing an analysis sample according to claim 1 or 2, wherein
the amidation reaction solution does not contain a dehydration-condensation agent to be reacted with the lactone.

6. The method for preparing an analysis sample according to claim 1 or 2, wherein
in the amidation reaction, after the sample is contacted with the amidation reaction solution, an operation of reacting the sample with a dehydration-condensation agent is not performed.

7. The method for preparing an analysis sample according to claim 1 or 2, wherein
a time during which the sample is in contact with the amidation reaction solution to perform the amidation reaction is shorter than 30 minutes.

8. The method for preparing an analysis sample according to claim 1 or 2, wherein
the first compound contains a linear hydrocarbon group.

9. The method for preparing an analysis sample according to claim 8, wherein the linear hydrocarbon group is an alkyl group.

10. The method for preparing an analysis sample according to claim 9, wherein a carbon number of the linear hydrocarbon group is any one of 1 to 6.

11. The method for preparing an analysis sample according to claim 1 or 2, wherein
the lactone is formed in sialic acids of the glycan.

12. The method for preparing an analysis sample according to claim 11, wherein the lactone is formed in at least one of α2,3-sialic acid, α2,8-sialic acid, and α2,9-sialic acid of the glycan.

13. The method for preparing an analysis sample according to claim 11, further comprising
contacting the sample prepared with a lactonization reaction solution containing a dehydration-condensation agent to be reacted with the sialic acids to lactonize at least a part of the sialic acids.

14. The method for preparing an analysis sample according to claim 13, wherein the lactonization reaction solution further contains a reactant to be linked to the sialic acids contained in the glycan, and
the reactant is different in mass from the first compound, and
the sample is contacted with the lactonization reaction solution, a part of the sialic acids is lactonized based on a linkage type of the sialic acids, and at least a part of the reactant is linked to another part of the sialic acids.

15. The method for preparing an analysis sample according to claim 14, wherein the sample is contacted with the lactonization reaction solution to lactonize α2,3-sialic acid, α2,8-sialic acid, or α2,9-sialic acid and to link a part of the reactant to α2,6-sialic acid.

16. An analysis method comprising:
preparing an analysis sample by the method for preparing an analysis sample according to any one of claims 1 to 15; and
analyzing the analysis sample prepared.

17. The analysis method according to claim 16, wherein
the sample prepared is analyzed by at least one of mass spectrometry and chromatography.

18. A kit for preparing an analysis sample used in the method for preparing an analysis sample according to any one of claims 1 to 15 or the analysis method according to claim 16 or 17.
